# EUROPEAN PATENT SPECIFICATION

(11) **EP 1 645 262 B1**
(45) Date of publication and mention of the grant of the patent: **03.09.2008**
(21) Application number: 05300796.9
(22) Date of filing: 05.10.2005
(51) Int. Cl.: A61H 39/02

(54) **Multifunctional koryo therapy apparatus**
Multifunktionelles Koryo Therapiegerät
Appareil multifonctionel de therapie Koryo

(30) Priority: 06.10.2004 KR 2004079335
(43) Date of publication of application: 12.04.2006
(73) Proprietor: Yoo, Tae Woo, 137-762 Seoul (KR)
(72) Inventor: Yoo, Tae Woo, 137-762 Seoul (KR)
(74) Representative: Michelet, Alain

(56) References cited:
- EP-A- 1 040 847
- DE-A1- 19 717 337
- US-A- 4 763 657
- US-A1- 2003 055 357
- US-B1- 6 267 721
- US-B1- 6 421 560
- US-B1- 6 425 764

## Description

### BACKGROUND OF THE INVENTION

### Field of the Invention

The present invention relates to a multifunctional Koryo hand therapy apparatus and a method thereof. More specifically, the invention relates to a multifunctional Koryo hand therapy apparatus and a method thereof, in which an important acupuncture point and medical treatment guide of the hand therapy, Yeoampa therapy, skin resistance measurement, and low frequency treatment are provided in response to a user's selection.

### Background of the Related Art

Recently, diseases can be cured using Seoam press-pellets, T press-pellets, Seoam moxibustion, magnetic acupuncture, electron beams, and the like other than acupuncture, so that the acupuncture is renamed hand Yeoampa therapy which is more extensive than simple acupuncture.

The hand Yeoampa therapy is a treatment based on vision, hearing, hand center stimulation, hand therapy acupuncture theory, and Yeoampa (thought-wave) operation. Such Yeoampa therapy is a method in which the cerebrum of a body feels and recognizes the correct theory and prescription of hand therapy acupuncture, and visual and auditory senses stimulate the cerebrum. The Yeoampa therapy using such cyber hand therapy acupuncture stimulates visual and auditory senses more intensely than normal Yeoampa therapy, so that the effect response is stronger. Specifically, the effect can be maximized by statically stimulating for the same period of time after dynamic stimulation, and the same effect can be observed in an upright sitting method or a reinforcing and reducing method.
However, such important acupuncture points of hand therapy must be confirmed through scrutinizing specialized books one by one. Accordingly, a person who has steadily studied important acupuncture points of hand therapy through specialized books may be familiar with the important acupuncture points. However, ordinary persons cannot steadily study hand therapy, so that the important acupuncture points and hand therapy corresponding to symptoms are difficult to understand.

Document DE-A-19717337 describes an apparatus for acupuncture.

### SUMMARY OF THE INVENTION

Therefore, the present invention has been made in view of the above problems, and it is an object of the present invention to provide a multifunctional Koryo hand therapy apparatus and a method thereof, which provide an important acupuncture point and medical treatment guide, perform Yeoampa therapy in connection with the important acupuncture point guide without using hand therapy acupuncture instruments, the Yeoampa therapy being a cyber treatment that controls the functions of a body, measure skin resistance, and perform low frequency treatment in response to a user's selection.

To accomplish the above object, according to one aspect of the present invention, there is provided a multifunctional Koryo hand therapy apparatus comprising a memory unit for storing the important acupuncture point and prescription guide information of the hand therapy, a key input unit for generating key signals according to the user's selection of skin resistance measurement, important acupuncture point and prescription guide of low frequency treatment hand therapy, and Yeoampa therapy, a skin resistance measuring unit for outputting current for measuring skin resistance according to the selection of the skin resistance measurement from the key input unit, and measuring the skin resistance of a body based on the result value, a low frequency current treatment unit for supplying a low frequency to the stimulation points of a body in order to mitigate pains through skin stimulation according to the selection of the low frequency treatment from the key input unit, a controller for extracting the corresponding information from the important acupuncture point and prescription guide information of the hand therapy stored in the memory unit according to the key signal generated by the key input unit, performing Yeoampa therapy by outputting a hand therapy acupuncture pattern for performing Yeoampa therapy, and controlling the operations of the skin resistance measuring unit and low frequency current treatment unit respectively, and a display unit for displaying the important acupuncture point and prescription guide information of the hand therapy outputted by the controller, and the hand therapy acupuncture pattern for performing Yeoampa therapy wherein, said apparatus has means for: while Yeoampa therapy is performed by the controller, the low frequency treatment unit applies a low frequency to parts of a body that correspond to the hand therapy acupuncture pattern for performing Yeoampa therapy according to the key signals outputted from the key input unit.

The skin resistance measuring unit preferably includes a measuring unit meter for flowing current to the measured part, and measuring skin resistance according to the amount of the current, a meridian measuring lead connecting port that functions as a port for connecting a skin resistance lead, a measuring unit meter controller for controlling the apparatus so that the value of the meter becomes a certain value by contacting the skin resistance leads, and a lamp that is lit in order to indicate the skin resistance measurement.

The low frequency current treatment unit includes a low frequency generator for generating a low frequency, a current intensity adjustor for adjusting the current intensity of the low frequency generated by the low frequency generator, a frequency adjustor for adjusting the low frequency generated by the low frequency generator to an appropriate frequency, a treatment lamp for turning on and off the lamp having the low frequency generated by the low frequency generator, a timer for setting an output time of the low frequency generated by the low frequency generator, a start key for generating a key signal for starting low frequency treatment within the time set by the timer, and a low frequency lead connecting port for connecting a low frequency lead.

The multifunctional Koryo hand therapy method comprises the steps of (i) storing the important acupuncture point and prescription guide information of the hand therapy, (ii) judging whether a user selects one of the skin resistance measurement, low frequency current treatment, or important acupuncture point and prescription guide of the hand therapy, (iii) outputting current for measuring skin resistance, and measuring the skin resistance of a body based on the result value in a case where the user selects the skin resistance measurement, supplying a low frequency to the stimulation points of a body, and performing low frequency current treatment in order to mitigate pains through skin stimulation in a case where the user selects the low frequency current treatment, and extracting and outputting the information corresponding to a desired menu from the stored important acupuncture point and prescription guide information of the hand therapy in a case where the user selects the important acupuncture point and prescription guide of the hand therapy, (iv) judging whether the user selects the Yeoampa therapy while the important acupuncture point and prescription guide information of the hand therapy is outputted, (v) outputting a hand therapy acupuncture pattern for performing Yeoampa therapy, and performing Yeoampa therapy in a case where the Yeoampa therapy is selected, (vi) judging whether the user selects the low frequency treatment while Yeoampa therapy is being performed, and (vii) applying a low frequency to the parts of a body that correspond to the hand therapy acupuncture pattern for Yeoampa therapy and performing Yeoampa therapy in a case where the low frequency treatment is selected.

Step (iii) preferably includes the steps of providing an important acupuncture point and prescription information guide screen of the hand therapy having a plurality of menus, judging whether a desired menu is selected from the important acupuncture point and prescription guide screen of the hand therapy, and, in a case where the user selected a desired menu, extracting corresponding information from the stored important acupuncture point and prescription guide information of the hand therapy, and displaying the extracted information.

### BRIEF DESCRIPTION OF THE DRAWINGS

The above and other objects, features and advantages of the present invention will be apparent from the following detailed description of the preferred embodiments of the invention in conjunction with the accompanying drawings, in which:

Fig. 1 is block diagram showing the configuration of a multifunctional Koryo hand therapy apparatus according to an embodiment of the invention;

Fig. 2 is a perspective view showing the multifunctional Koryo hand therapy apparatus depicted in Fig. 1;

Fig. 3 is an front view showing the multifunctional Koryo hand therapy apparatus depicted in Fig. 2;

Fig. 4 is a plain view showing a remote controller according to an embodiment of the invention;

Fig. 5 shows a television set connected to the multifunctional Koryo hand therapy apparatus in Fig. 2;

Figs. 6 and 7 show the screens that provide an important acupuncture point and medical treatment guide of Koryo hand therapy according to an embodiment of the invention; and

Fig. 8 shows a flowchart explaining a multifunctional Koryo hand therapy method according to an embodiment of the invention.

### DETAILED DESCRIPTION OF THE PREFERRED EMBODIMENT

The preferred embodiments of the invention will be hereafter described in detail, with reference to the accompanying drawings.

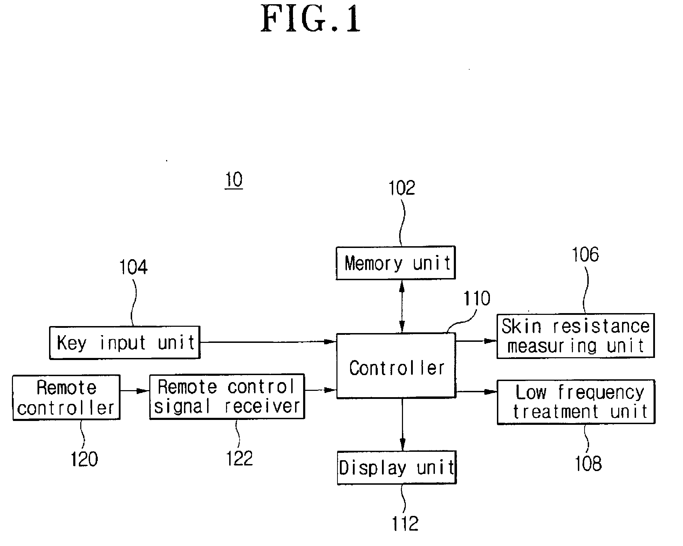

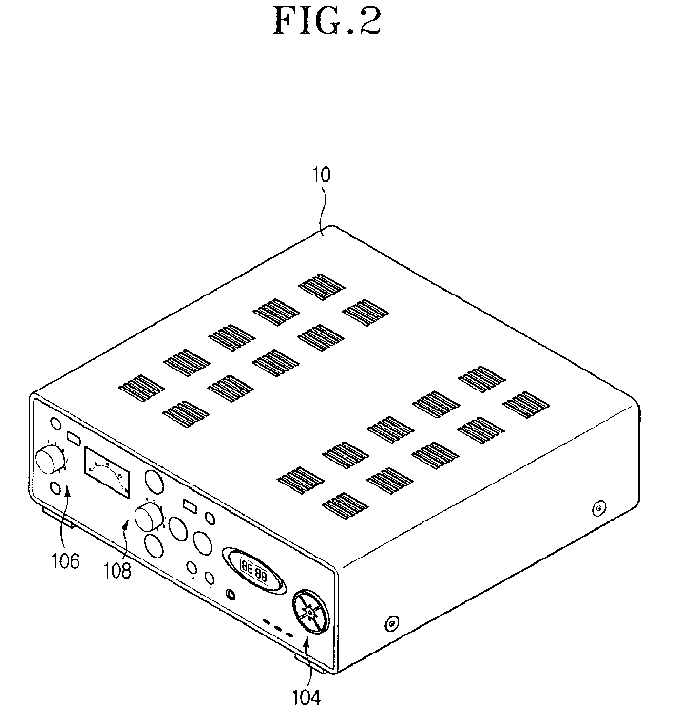

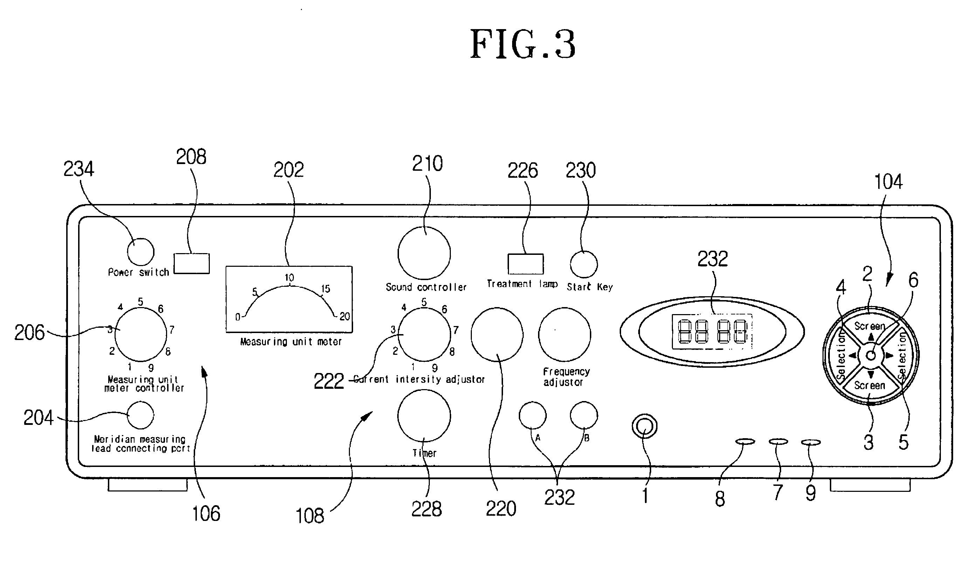

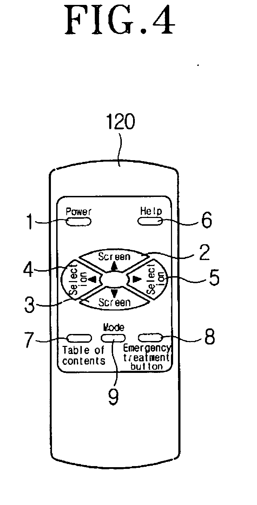

Fig. 1 is block diagram showing the configuration of a multifunctional Koryo hand therapy apparatus according to an embodiment of the invention. Fig. 2 is a perspective view showing the multifunctional Koryo hand therapy apparatus depicted in Fig. 1. Fig. 3 is a front view showing the multifunctional Koryo hand therapy apparatus depicted in Fig. 2. Fig. 4 is a plain view showing a remote controller according to an embodiment of the invention.

The multifunctional Koryo hand therapy apparatus 10 according to an embodiment of the invention includes a memory unit 102, key input unit 104, skin resistance measuring unit 106, low frequency treatment unit 108, controller 110, and display unit 112. The multifunctional Koryo hand therapy apparatus 10 further includes a remote controller 120 for generating remote control signals according to the user's selection of the important acupuncture point guide of the hand therapy, Yeoampa therapy, skin resistance measurement, and low frequency current treatment, and a remote control signal receiver 122 for receiving remote control signals from the remote controller 120. As shown in Fig. 3, the multifunctional Koryo hand therapy apparatus 10 further includes a light emitting diode 232 for displaying the skin resistance measured by the skin resistance measuring unit 106, a sound controller 210 for generating a high-pitched sound at the peak point when the skin resistance measuring unit 106 is in operation, and generating and controlling the sound corresponding to the low frequency current generated by the low frequency treatment unit 108, and a main power switch 234 for selecting the skin resistance measurement or low frequency current treatment.

The memory unit 102 stores the important acupuncture point and prescription guide information of the hand therapy.

The key input unit 104 generates key signals according to a user's selection of the skin resistance measurement, important acupuncture point and prescription guide of low frequency current treatment, and Yeoampa therapy. A power button 1 is used when the user turns on or off the power in order to use an important acupuncture point guiding device of hand therapy according to the invention.

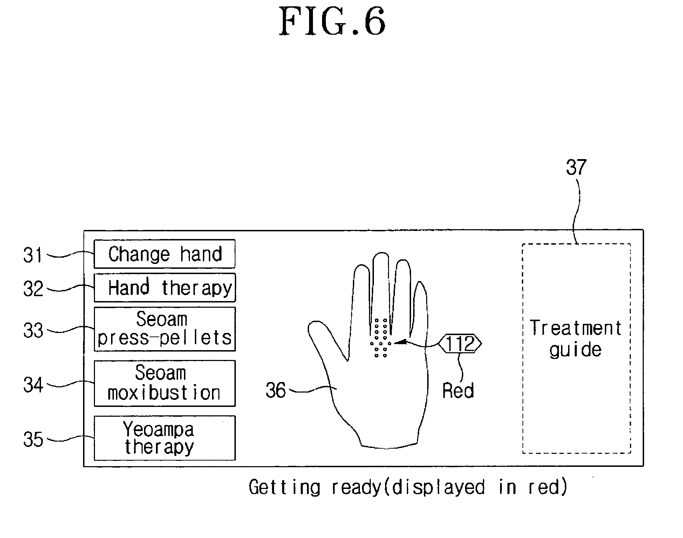

The up ▲ button 2 is a button for selecting a symptom, which is used to switch screens. If the button is pressed once when the apparatus is used for Yeoampa therapy, a red acupuncture pattern 112 is displayed as shown in Fig. 6, and the apparatus is in a ready state for Yeoampa therapy. It the button 2 is pressed once more, Yeoampa therapy is performed. (In order to release the Yeoampa therapy, press the button once more. In addition, the button is pressed in order to switch to a screen showing the palm or back of a hand among prescription screens (including Yeoampa therapy, important acupuncture points, 14 micro-meridians), and all the prescription screens must be followed by a screen displayed to confirm prescription points.

The down V button 3 is a button for releasing the symptom screen, which performs a counter function to the up ▲ button 2, and is used when a screen moves back to the previous screen. In addition, it is used when the Yeoampa therapy is completed, and the apparatus is released from the Yeoampa therapy.

The left selection button 4 is a button for selecting a symptom that a user desires to find from the list displayed by handling the up ▲ and down ▼ buttons 2 and 3, which moves a pointer to the left or up according to the arranged list. In addition, it is also used to move an acupuncture point while acupuncture point descriptions are displayed or Yeoampa therapy is performed.

The right selection button 5 is a button for selecting a symptom that a user desires to find from the list displayed by handling the up ▲ and down ▼ buttons 2 and 3, which moves a pointer to the right or down according to the arranged list. In addition, it is also used to move an acupuncture point while acupuncture point descriptions are displayed or Yeoampa therapy is performed.

The help button 6 is used when the symbols or exact locations of acupuncture points, and detailed descriptions on acupuncture points are needed while a prescription screen is used. Also, when a help function is released, the help button 6 is pressed once. However, when the location of an acupuncture point is moved, the left and right selection buttons 4 and 5 are used. In addition, when reinforcing and reducing stimulation is performed while Yeoampa therapy is performed, this button 6 moves the acupuncture pattern.

The contents button 7 is used when a screen is moved to the initial contents while a prescription screen is selected and used.

The emergency prescription button 8 is used when an emergency situation arises, or any screen that is being used is directly switched to the emergency prescription contents screen. According to an embodiment of the invention, as shown in Fig. 3, the hand therapy apparatus is preferably handled through a remote controller 120. However, the key input unit 104 depicted in Fig. 2 can be used to handle the apparatus.

The skin resistance measuring unit 106 outputs current for measuring skin resistance according to the selection of the skin resistance measurement from the key input unit 104, and measures the skin resistance of a body based on the result value. The skin resistance measuring unit 106 flows current through the hands and feet that are connected to the meridian points of the five viscera and six bowels (12 intestines) in a body. In this case, if the current flowing through the hands and feet is significantly more or less than that of a normal state, the skin resistance measuring unit 106 measures such a response and confirms whether any intestine has a disorder. Specifically, the skin resistance measuring unit 106 is a means that measures the skin resistances appearing at the meridian points of six bowels (stomach, bladder, gall, triple-warmer, small intestine, large intestine) of the left and right hands, and six viscera (spleen, lung, liver, kidney, kidney, heart) of the left and right feet.

The skin resistance measuring unit 106 includes a measuring unit meter 202, a meridian measuring lead connecting port 204, a measuring unit meter controller 206, and a lamp 208.

The measuring unit meter 202 flows current to the measured part, and measures skin resistance based on the amount of the current. The meridian measuring lead connecting port 204 functions as a port for connecting skin resistance leads. The measuring unit meter controller 206 controls the apparatus so that the value of the meter becomes a certain value by contacting the skin resistance leads, preferably 200 µA. The lamp 208 is lit in order to indicate that the skin resistance measurement is in operation.

The method of measuring skin resistance by the skin resistance measuring unit 906 configured as above is explained below.

First, a skin resistance lead is connected to the meridian measuring lead connecting port 204.

The main power switch 234 is turned down toward the skin resistance measurement, and the lamp 208 is turned on A probe lead is contacted with an opposite lead (a handle lead), and the measuring unit meter controller 206 is adjusted so that the pointing value of the measuring unit meter 202 becomes 200 µA. Holding the opposite lead and contacting the probe lead to the skin, a user diagnoses symptoms using the numerals of the measuring unit meter 202 that are recorded on a graph paper. When the pointing value of the measuring unit meter 202 arrives at the peak value, a high-pitched sound is generated through a speaker, and when the sound is not needed, the sound controller 210 is turned anti-clockwise.

According to the selection of the low frequency current treatment from the key input unit 104, the low frequency current treatment unit 108 supplies a low frequency current to the stimulation points of a body in order to mitigate pains through skin stimulation The low frequency treatment unit 108 is widely used to stimulate stimulation points of a body using a low frequency, and thus to prevent or treat various kinds of diseases. By relaxing muscles and improving blood circulation, the health of a body can be improved.

The low frequency current treatment unit 108 includes a low frequency generator 220, current intensity adjustor 222, frequency adjustor 224, treatment lamp 226, timer 228, start key 230, and low frequency lead connecting port 232.

The low frequency generator 220 generates a low frequency. The current intensity adjustor 222 adjusts the current intensity of the low frequency generated by the low frequency generator 220. The frequency adjustor 224 adjusts the low frequency generated by the low frequency generator 220 to a frequency that a user expects. The treatment lamp 226 turns on and off the lamp having the low frequency generated by the low frequency generator 220. The timer 228 sets an output time of the low frequency generated by the low frequency generator 220. The start key 230 generates a key signal for starting low frequency treatment within the time set by the timer 226. The low frequency lead connecting port 232 is a two-channel connecting port that connects low frequency leads.

In a method using such a low frequency current treatment unit described above, first, a low frequency current treatment lead is connected to the low frequency current lead connecting port 232. The main power switch 234 is switched up toward the low frequency treatment position. The current intensity adjustor 222 and frequency adjustor 224 are adjusted so as to appropriately stimulate a patient. In addition, the timer 228 can adjust the period of time for appropriate treatment. For example, the timer 228 can adjust the operation time of low frequency treatment to 0-30 seconds. The apparatus starts to be used by pressing the start key 230, and after the adjusted time is elapsed, the operation automatically stops. In this way, the output of the low frequency more than needed is controlled, so that a user can safely use the apparatus. A sound corresponding to a frequency is generated through the speaker, and when the sound is not desired, the sound controller 210 is turned to the counterclockwise. After treatment is completed, if the treatment is required again, the start key 230 is pressed.

According to the key signal generated by the key input unit 104, the controller 110 extracts corresponding information from the important acupuncture point and prescription guide information of the hand therapy stored in the memory unit 102. The controller 110 outputs a hand therapy acupuncture pattern for performing Yeoampa therapy, and performs Yeoampa therapy. The controller 110 controls the operations of the skin resistance measuring unit 106 and low frequency current treatment unit 108 respectively. While Yeoampa therapy is performed by the controller 110, according to the key signals outputted from the key input unit 104 or handling of the main power switch 234, the low frequency current treatment unit 108 applies a low frequency current to the parts of a body that correspond to the hand therapy acupuncture pattern for performing Yeoampa therapy.

The display unit 112 displays the important acupuncture point and prescription guide information of the hand therapy outputted by the controller 110, and the hand therapy acupuncture pattern for performing Yeoampa therapy.

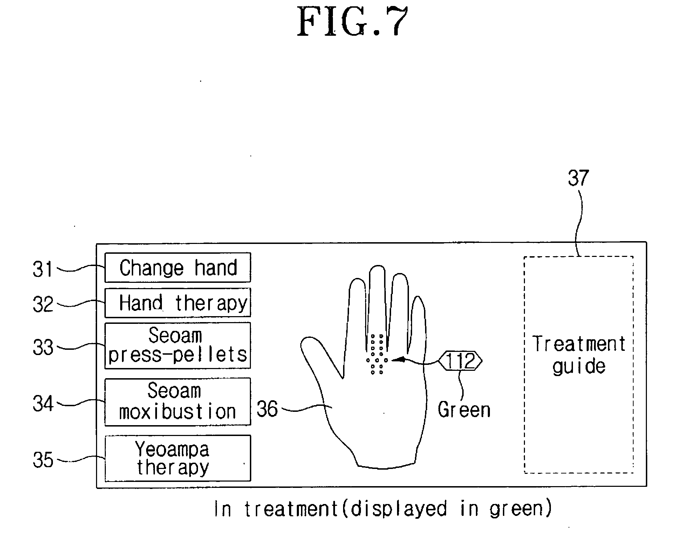

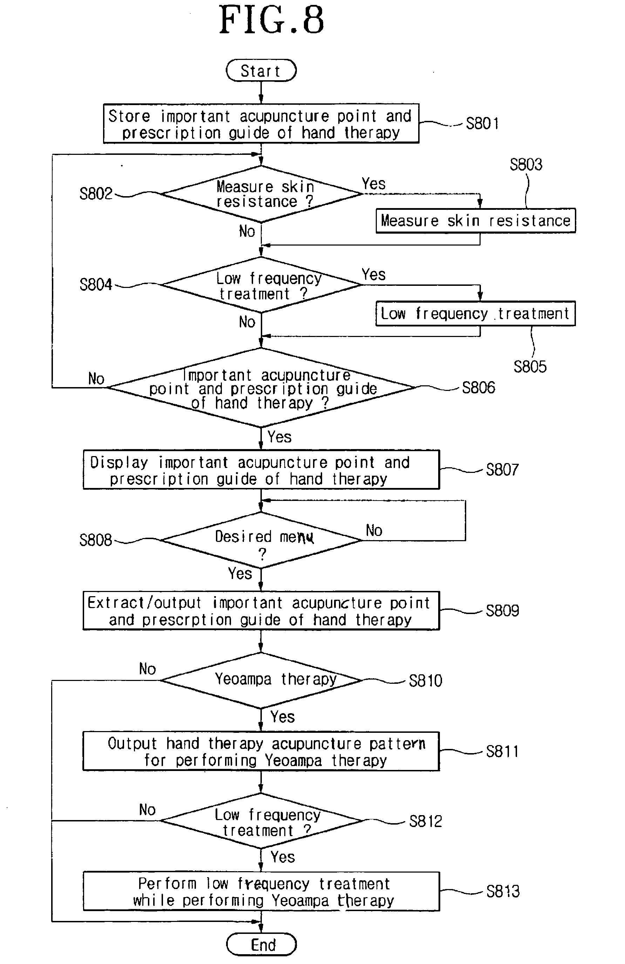

Hereafter, a method of the multifunctional Koryo hand therapy according to an embodiment of the invention is explained with reference to Figs. 6 to 8. Figs. 6 and 7 show the screens that provide the important acupuncture point and medical treatment guide of Koryo hand therapy according to an embodiment of the invention. Fig. 8 shows a flowchart explaining a multifunctional Koryo hand therapy method according to an embodiment of the invention.

The controller 110 stores the important acupuncture point and prescription guide information of the hand therapy in the memory unit 102 (step S801 ).

The controller 110 judges whether a user handled the main power switch 234, and selected the skin resistance measurement (step S802).

As a result of the judgment in step S802, in a case where the user selects the skin resistance measurement, the skin resistance measuring unit 106 outputs current for measuring skin resistance under the control of the controller 110, and measures the skin resistance of a body based on the result value (step S803). Alternatively, in a case where the user does not select the skin resistance measurement, the controller 110 judges whether the user handled the main power switch 234, and selected the low frequency treatment (step S804).

As a result of the judgment in step S804, in a case where the user selects the low frequency current treatment, the controller 110 controls the low frequency current treatment unit 108 so as to supply a low frequency current to the stimulation points of a body, and performs low frequency current treatment in order to mitigate pains through skin stimulation (step S805). Alternatively, as a result of the judgment in step S804, in a case where the user does not select the low frequency current treatment, the controller 110 judges whether the important acupuncture point and prescription guide of the hand therapy is selected (step S806).

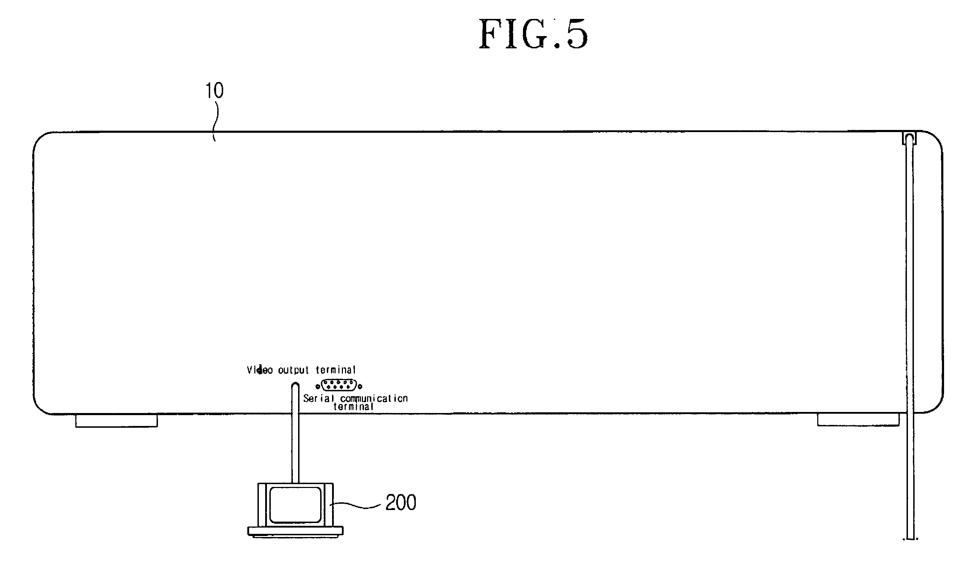

As a result of the judgment in step S806, in a case where the user selects the important acupuncture point and prescription guide of the hand therapy, the controller 110 provides an important acupuncture point and prescription guide screen of the hand therapy having a plurality of menu items in response to the user's selection (step S807). Fig. 5 shows a television set connected to the multifunctional Koryo hand therapy apparatus in Fig. 2. Referring to Fig. 5, a video connection cable is inserted into the video output terminal of the multifunctional Koryo hand therapy apparatus according to the invention, and is connected to the video input terminal of a television set 200. At this point, in a case where the television set has a plurality of external input terminals, a 'video' or 'video input' terminal must be selected to connect. After connecting the television set as shown in Fig. 5, the power of the television set 200 is turned on, and the power button 1 of the key input unit 104 or the remote controller 120 of the invention is pressed so that the power of the hand therapy apparatus is turned on. At this point, in the case of a television set 220 having a pair of terminals (audio, video), the 'TV/Video' button of the remote controller 120 is switched so that the television set 200 is switched to the video mode. On the other hand, in the case of a television set 220 having a plurality pairs of video terminals (audio, video), the 'external input' selection button of the remote controller 120 is selected in order to set to the 'external input' terminal that is previously connected.

Through the operations described above, the initial screen of the important acupuncture point guide apparatus of hand therapy according to the invention is displayed, and the apparatus is ready to be used.

In step S808, the controller 110 judges whether or not the user selected a desired menu from the important acupuncture point and prescription guide screen the of hand therapy.

As a result of the judgment in step S808, in a case where the user selected a desired menu, the controller 110 extracts the information corresponding to the selected menu from the stored important acupuncture point and prescription guide information of the hand therapy, and outputs the extracted information through the display unit 112 (step S809).

For example, in the initial screen state, the user presses the screen up ▲ button 2 of the remote controller 120.

Then, a selection contents screen configured with "Where do you feel the pain?", ''Who is sick?", "Hand therapy acupuncture is...", "Emergency prescription", and "Cosmetic/Health" is provided on the screen.

At this point, in a case where the user selects "Where do you feel the pain?", as shown in Table 1, the user can select the symptom of a painful part that is categorized by the part of a body.

**[Table 1]**

| Items | Parts | Contents |
|---|---|---|
| What is your problem? | Head | chronic headache, anemia, stress, foreheadache, carsickness, migraine, etc. |
| | Ear | tinnitus, ear pain, etc. |
| | Mouth | stomatitis, herpetic stomatitis, toothache, toothache due to wind, etc. |
| | Shoulder | armpit pain, collarbone pain, stiff shoulder, etc. |
| | Abdomen | hepatitis, irritable colon symptom, acute gastro enteritis, cystitis, constipation, dyspepsia, food poisoning, etc. |
| | Back | back pain, thoracic vertebrae pain, etc. |
| | Genital organs | impotence, frigidity, leucorrhea decline, sexual dysfunction, urethritis, etc. |
| | Arm | hand wind, toe, tinea pedis, hand numbness and clamp, housewife's eczema, etc. |
| | Leg | knee arthralgia, athlete's foot, foot pain, groin eczema, calf pain, etc. |
| | Eye | conjunctivitis, presbyopia, eye fatigue, tear, sty, amblyopia, etc. |
| | Nose | splenitis, empyema, snoring, nasal congestion, nasal hemorrhage, rhinorrhea cold, etc. |
| | Throat | cough and phlegm, chronic bronchitis, hoarseness, pharyngitis, tonsillitis etc. |
| | Waist | tailbone pain, hypertension, hypotension, rib pain, mother milk decline, Breast disease, etc. |
| | Anus | anal fistula, hemorrhoids, anus prolapse, anal hemorrhage |
| | Womb | internal hectic fever, leukorrhea, infertilily, menstrual pain, recurrent pregnancy loss, menstrual disorder, vaginitis, etc. |
| | Skin | melasma , freckle, urticaria, dermatitis, Osmidrosis, pimple, etc. |
| | Chest | rib pain, hypertension, hypotension, rib pain, mother milk decline, Breast disease, etc. |

On the other hand, in a case where the user selects "Who is sick?", as shown in Table 2, the contents of the program can be categorized by the type of a user who is in need, such as an examinee, housekeeper, employee, or the like, and be used to meet the user's purposes.

**[Table 2]**

| Table of contents | Class | Contents |
|---|---|---|
| Who is sick? | examinee | clearing eyes and head, epistaxis, amblyopia, fatigue prevention, stiff shoulder, etc. |
| | housekeeper | mother milk decline, infertility, menstrual irregularity and pain, housewife's eczema, making glossy skin, etc. |
| | employee | strengthening liver function, hangover settlement, halitosis, chronic fatigue, indigestion, stress settlement, etc. |
| | senior | dark spots, facial semi-paralysis, bronchial asthma, knee arthralgia, cataract, etc. |
| | infant | acute convulsion, hiccupping, baby pyrexia, bedwetting, jaundice, burning, etc. |
| | skin care | developing chest, removing extra flesh, crow's feet, constipation, pimple, melasma and freckle, etc. |
| | diet | removing extra flesh, obesity, weight control, heart care while dieting, improving intestinal function, etc. |
| | quit smoking | quit smoking |
| | sound sleep | sound sleep |
| | athlete's foot | tinea manus and tinea pedis, heel keratin, toenail deformation, groin eczema, etc. |
| | eyesight | strengthening liver function, conjunctivitis, acute glaucoma, presbyopia, amblyopia, etc. |
| | loss of hair | alopecia areata, alopecia, etc. |

In addition, in a case where the user selects "Hand therapy acupuncture is...", as shown in Table 3, introductions to the cyber hand therapy acupuncture and descriptions of the instruments, correspondence therapy, 14 micro-meridians and important acupuncture points (source point, eight extraordinary points, etc.), and the information related to hand therapy acupuncture are displayed.

**[Table 3]**

| Items | Menu | Contents |
|---|---|---|
| Hand therapy acupuncture is... | Cyber hand therapy | Introducing history of cyber hand therapy, and outline and strength of hand therapy |
| | Hand therapy instruments and instructions | Seoam moxibustion, Seoam press-pellets, Seoam ring, bloodletting needle, cupping glass, etc. |
| | Hand therapy correspondence map | Front and backside of a body, etc. |
| | Hand therapy 14 micro-meridians and important acupuncture points | Introducing 14 micro-meridians and 345 acupuncture points Introducing important acupuncture point (Twelve Source Point, Well point, Five Shu, Front Mo, Back Shu, Eight Extraordinary points) |

In addition, in a case where the user selects "Emergency prescription", as shown in Table 4, the screen is switched to the emergency prescription contents, and is immediately used. The items of the emergency prescription contents are infant acute convulsion, senseless, unconsciousness, shock, chest pain, food poisoning, fever, nasal hemorrhage, etc.

**[Table 4]**

| Items | Contents |
|---|---|
| Emergency treatment | infant acute convulsion, senseless, unconsciousness, shock, chest pain, food poisoning, fever, nasal hemorrhage, weak people shock treatment, etc. |
| Cosmetic/Health | removing extra flesh, obesity, unbalanced diet in meat, clearing eyes, alleviating parturition pain, women's health care, improving stamina, preventing and controlling wind stroke, fatigue in spring, etc. |

In addition, in a case where the user selects "Cosmetic/Health", as shown in Table 4, the screen is switched to the Cosmetic/Health contents.

Here, while using the prescription contents, if a command (change hands, hand therapy acupuncture, Seoam moxibustion, and Yeoampa therapy) placed at the left side of the screen is selected using the left and right selection buttons 4 and 5, and assigned using the screen up ▲ button 2, the points on the palm and back of a hand can be confirmed in detail, and the screen can be switched to the detailed descriptions of basic treatment instruments or Yeoampa therapy.

Second, at this point, in a case where a user is assumed to have a chronic headache, "Where do you feel the pain?" is selected by pressing the left and right selection buttons 4 and 5, and the screen up A button 2 is pressed.

Third, since the chronic headache corresponds to the head part, the head part shown in Table 1 is pointed, and the screen up A button 2 is pressed.

Fourth, if the chronic headache of the head part in Table 1 is pointed, and the screen up A button 2 is pressed, the important acupuncture point and hand therapy acupuncture prescription guide 37 based on a palm shape 36 is displayed as shown in Fig. 6 or 7.

Fifth, the user performs treatment following the prescription guide 37 that is displayed at the right side of the screen, and presses the help button 6 if the location or description of each acupuncture point is needed. In a case where the description of an acupuncture points is further desired, the left and right selection buttons 4 and 5 are pressed. Then, in the case of releasing the help function, the help button 6 is pressed once more.

Sixth, prescription types, such as hand therapy acupuncture (acupuncture, moxibustion) 32, Seoam press-pellets 33, Seoam moxibustion 34, Yeoampa therapy 35, and the like, are provided at the left side of the screen. If the user desires to use the Yeoampa therapy 35, the screen up ▲ button 2 is pressed after pointing to the Yeoampa therapy using the left and right selection buttons 4 and 5.

Seventh, the user presses and holds the screen down V button 3, or presses the contents button 7 in order to return to the initial screen.

While the important acupuncture point and prescription guide information of the hand therapy is outputted, the controller 110 judges whether the user presses the mode key 9, and selected the Yeoampa therapy 35 (step S810).

As a result of the judgment in step S810, in a case where the Yeoampa therapy 35 is selected, the controller 110 outputs a hand therapy acupuncture pattern for performing Yeoampa therapy through the display unit 112, and performs Yeoampa therapy.

In the embodiment of the invention, Yeoampa therapy can be applied to the conventional hand therapy acupuncture prescription and correspondence map, 14 micro-meridians and important acupuncture points, and the like. In a case where the hand therapy prescription is Yeoampa stimulation, when Yeoampa therapy is to be performed, the screen is moved to Yeoampa therapy contents using the left and right selection buttons 4, 5.

If the screen up ▲ button 2 is pressed, as shown in Fig. 6, a red hand therapy acupuncture pattern is displayed, and, at this point, Yeoampa therapy is ready to be performed.

If the screen up ▲ button 2 is pressed once more, as shown in Fig. 7, the hand therapy acupuncture pattern is changed to green, and Yeoampa therapy is performed.

When releasing Yeoampa therapy, if the screen up ▲ button 2 is pressed once more, the hand therapy acupuncture pattern is changed to red, and Yeoampa therapy is released.

On the other hand, when acupuncture points are to be changed, Yeoampa therapy is released in the way described above, and the left and right selection buttons 4 and 5 are pressed in order to move to the desired acupuncture point.

In addition, when the reinforcing and reducing method according to the invention is performed, if the help button 6 is pressed while Yeoampa therapy is being performed, the needle moves from bottom to the top in order, and the reinforcing and reducing stimulation of Yeoampa therapy is performed by pressing the right selection buttons 5.

In addition, in a case where Yeoampa therapy is performed on the 14 micro-meridians and important acupuncture points (Twelve Source Point, Well point, Five Shu, Front Mo, Back Shu, Eight Extraordinary points), on the initial screen of the important acupuncture point guiding device of the hand therapy according to the invention, 'Hand therapy acupuncture is ...', hand therapy, 14 micro-meridians and important acupuncture points, and important acupuncture points are selected in order using the left and right selection buttons 4 and 5, and the screen up A button 2 is pressed.

Next, a desired item among the Twelve Source Point, Well point, Five Shu point, Front Mo point, Back Shu point, and Eight Extraordinary points of the important acupuncture points is pointed using the left and right selection buttons 4 and 5, and the screen up A button 2 is pressed.

Next, a desired item among the Change hand, Hand therapy acupuncture, Seoam press-pellets, Seoam moxibustion, and Yeoampa therapy displayed at the left side of the screen is pointed using the left and right selection buttons 4 and 5, and the screen up A button 2 is pressed.

Next, since Yeoampa stimulation is designed to stimulate the acupuncture points on the right hand first, if the screen up A button 2 is pressed, the acupuncture points on the right hand are stimulated, and then if the left selection buttons 4 is pressed, the screen is switched to the left hand screen, and then the screen up ▲ button 2 is pressed to perform Yeoampa therapy.

At this point, when reinforcing and reducing stimulation is performed through Yeoampa therapy, the acupuncture pattern indicating a Yeoampa halt state is red. At this point, the locations of reinforcing and reducing is determined by pressing the help button 6, and also the screen up A button 2 is pressed to performed Yeoampa stimulation.

Next, after the completion of the Yeoampa stimulation, the screen down ▼ button 3 is pressed and hold, or the contents button 7 is pressed in order to return to the initial screen.

In step S812, while Yeoampa therapy is performed, the controller 110 judges whether the user handles the main power switch 234, and selects the low frequency treatment.

As a result of the judgment in step S812, in a case where the low frequency current treatment is selected, the low frequency current treatment unit 108 applies a low frequency current to the parts of a body that correspond to the hand therapy acupuncture pattern for Yeoampa therapy under the control of the controller 110, and thus Yeoampa therapy is performed (step S813).

As explained above, according to the invention, skin resistance measurement, low frequency current treatment, important acupuncture point and prescription information guide of the hand therapy, and Yeoampa therapy can be performed according to a user's selection.

In guiding the important acupuncture point and prescription information of the hand therapy, the important acupuncture point and prescription information guide of the hand therapy is stored in the memory unit, and the correct important acupuncture point and treatment guide information of the hand therapy can be provided in the form of a static image through the display unit according to a user's symptom or status. Accordingly, people in their prime or old ages or employees can perform hand therapy or Yeoampa therapy by themselves using the important acupuncture points provided by the multifunctional hand therapy apparatus according to the invention. In addition, while Yeoampa therapy is being performed, low frequency current is applied to the parts of a body that correspond to the hand therapy acupuncture pattern for performing Yeoampa therapy, and low frequency current treatment can be performed at the same time.

While the present invention has been described with reference to the particular illustrative embodiments, it is not to be restricted by the embodiments but only by the appended claims. It is to be appreciated that those skilled in the art can change or modify the embodiments without departing from the scope of the present invention. For example, the invention can be applied to an HHP, CT-2, cellular phone, digital phone, PCS phone, PDA, or mobile terminal.

## Claims

1. A multifunctional Koryo hand therapy apparatus comprising:
a memory unit for storing important acupuncture point and prescription guide information of the hand therapy;
a key input unit for generating key signals according to a user's selection of skin resistance measurement, important acupuncture point and prescription guide of low frequency treatment hand therapy, and Yeoampa therapy;
a skin resistance measuring unit for outputting current for measuring skin resistance, according to the selection of the skin resistance measurement from the key input unit, and measuring the skin resistance of a body based on a result value;
a low frequency current treatment unit for supplying a low frequency to stimulation points of a body in order to mitigate pains through skin stimulation, according to the selection of the low frequency treatment from the key input unit;
a controller for extracting corresponding information from the important acupuncture point and prescription guide information of the hand therapy stored in the memory unit according to the key signal generated by the key input unit, performing Yeoampa therapy by outputting a hand therapy acupuncture pattern for performing Yeoampa therapy, and controlling operations of the skin resistance measuring unit and low frequency current treatment unit respectively; and
a display unit for displaying the important acupuncture point and prescription guide information of the hand therapy outputted by the controller, and the hand therapy acupuncture pattern for performing Yeoampa therapy,
wherein, while Yeoampa therapy is performed by the controller, the low frequency current treatment unit applies a low frequency current to parts of a body that correspond to the hand therapy acupuncture pattern for performing Yeoampa therapy according to the key signals outputted from the key input unit.

2. The apparatus according to claim 1, wherein the skin resistance measuring unit includes:
a measuring unit meter for flowing current to a measured part, and measuring the skin resistance according to amount of the current;
a meridian measuring lead connecting port that functions as a port for connecting a skin resistance lead;
a measuring unit meter controller for controlling the apparatus so that a value of the meter becomes a certain value by contacting the skin resistance leads; and
a lamp that is lit in order to indicate the skin resistance measurement.

3. The apparatus according to claim 1, wherein the low frequency current treatment unit includes:
a low frequency generator for generating a low frequency;
a current intensity adjustor for adjusting current intensity of the low frequency generated by the low frequency generator;
a frequency adjustor for adjusting the low frequency generated by the low frequency generator to an appropriate frequency;
a treatment lamp for turning on and off the lamp having the low frequency generated by the low frequency generator;
a timer for setting an output time of the low frequency generated by the low frequency generator;
a start key for generating a key signal for starting low frequency treatment within the time set by the timer; and
a low frequency lead connecting port for connecting a low frequency lead.

4. The apparatus according to claim 1, further comprising:
a remote controller for generating remote control signals according to the user's selection of the important acupuncture point guide of the hand therapy, Yeoampa therapy, skin resistance measurement, and low frequency current treatment; and
a remote control signal receiver for receiving remote control signals from the remote controller.

5. The apparatus according to claim 1, further comprising:
a light emitting diode for displaying the skin resistance measured by the skin resistance measuring unit.

6. The apparatus according to claim 1, further comprising:
a sound controller for generating a high-pitched sound at a peak point when the skin resistance measuring unit is in operation, and generating and controlling a sound corresponding to the low frequency generated by the low frequency current treatment unit.

7. The apparatus according to claim 1, further comprising:
a main power switch for selecting the skin resistance measurement or low frequency current treatment.

## Patentansprüche

1. Multifunktionelles Koryo-Handtherapiegerät, das
eine Speichereinheit zur Speicherung wesentlicher Akupunkturpunkt- und Rezeptvorgabeinformationen der Handtherapie,
eine Tasten-Eingabeeinheit zur Erzeugung von Tastensignalen nach Wahl des Benutzers von Hautwiderstandsmessung, wesentlichen Akupunkturpunkt- und Rezeptvorgabeinformationen der Niederfrequenzbehandlungs-Handtherapie sowie der Yeoampa-Therapie,
eine Hautwiderstands-Messeinheit zur Ausgabe des Stroms zur Messung des Hautwiderstands entsprechend der Wahl der Hautwiderstandsmessung durch die Tasten-Eingabeeinheit und Messung des Hautwiderstands eines Körpers auf der Grundlage eines Ergebniswerts,
eine Niederfrequenzstrom-Behandlungseinheit zur Abgabe einer Niederfrequenz an Reizpunkte eines Körpers zur Mitigation von Schmerzen auf Grund der Hautreizung entsprechend der Wahl der Niederfrequenzbehandlung der Tasten-Eingabeeinheit,
ein Steuergerät zur Erfassung entsprechender Informationen aus den in der Speichereinheit gespeicherten wesentlichen Akupunkturpunkt- und Rezeptvorgabeinformationen der Handtherapie entsprechend dem durch die Tasteneingabeeinheit erzeugten Tastensignal, Durchführung der Yeoampa-Therapie durch Abgabe eines Handtherapie-Akupunkturmusters zur Durchführung der Yeoampa-Therapie und Steuerung des Betriebs der Hautwiderstands-Messeinheit bzw. der Niederfrequenzstrom-Behandlungseinheit, sowie
eine Anzeigeeinheit zur Anzeige der durch das Steuergerät abgegebenen wesentlichen Akupunkturpunkt- und Rezeptvorgabeinformationen der Handtherapie und des Handtherapie-Akupunktur-musters zur Durchführung der Yeoampa-Therapie,
wobei, während die Yeoampa-Therapie durch das Steuergerät durchgeführt wird, die Niederfrequenzstrom-Behandlungseinheit den Teilen eines Körpers einen Niederfrequenzstrom zuführt, die dem Handtherapie-Akupunkturmuster zur Durchführung der Yeoampa-Theorie gemäß den von der Tasten-Eingabeeinheit abgegebenen Tastensignalen entsprechen, umfasst.

2. Gerät nach Anspruch 1, wobei die Hautwiderstands-Messeinheit
ein Messeinheits-Messgerät für den Stromfluss zu einem gemessenen Teil und zur Messung des Hautwiderstands entsprechend dem Strombetrag,
einen meridianen Messleitungsverbindungskontakt, der als Kontakt zum Anschluss einer Hautwiderstandsleitung wirkt,
eine Messeinheits-Messgerätsteuervorrichtung zur Steuerung des Geräts, so dass ein Wert des Messgeräts durch Kontakt mit den Hautwiderstandsleitungen ein bestimmter Wert wird, sowie
eine Lampe, die zur Anzeige der Hautwiderstandsmessung aufleuchtet, umfasst.

3. Gerät nach Anspruch 1, wobei die Niederfrequenzstrom-Behandlungseinheit
einen Niederfrequenzgenerator zur Erzeugung einer Niederfrequenz,
eine Stromstärkeeinstellvorrichtung zur Einstellung der Stromstärke der durch den Niederfrequenzgenerator erzeugten Niederfrequenz,
eine Frequenzeinstellvorrichtung zur Einstellung der durch den Niederfrequenzgenerator erzeugten Niederfrequenz auf eine geeignete Frequenz,
eine Behandlungslampe zum Ein- und Ausschalten der Lampe mit der durch den Niederfrequenzgenerator erzeugten Niederfrequenz,
einen Zeitschalter zur Einstellung einer Abgabezeit der durch den Niederfrequenzgenerator erzeugten Niederfrequenz,
eine Starttaste zur Erzeugung eines Tastensignals zum Starten der Niederfrequenzbehandlung innerhalb der durch den Zeitschalter vorgegebenen Zeit, sowie
einen Niederfrequenzleitungs-Verbindungskontakt zum Anschluss einer Niederfrequenzleitung umfasst.

4. Gerät nach Anspruch 1, das weiterhin
eine Fernsteuerungsvorrichtung zur Erzeugung von Fernsteuerungssignalen nach Wahl der wesentlichen Akupunkturpunktvorgaben der Handtherapie durch den Benutzer, der Yeoampa-Therapie, der Hautwiderstandsmessung und der Niederfrequenzstrombehandlung, sowie
einen Fernsteuerungssignalempfänger zum Empfang der Fernsteuerungssignale von der Fernsteuerungsvorrichtung umfasst.

5. Gerät nach Anspruch 1, das weiterhin
eine LED zur Anzeige des durch die Hautwiderstands-Messeinheit gemessenen Hautwiderstands umfasst.

6. Gerät nach Anspruch 1, das weiterhin
eine Tonsteuerungsvorrichtung zur Erzeugung eines helltönenden Tons bei einem Spitzenpunkt, wenn die Hautwiderstands-Messeinheit in Betrieb ist, sowie zur Erzeugung und Steuerung eines Tons, der der durch die Niederfrequenzstrom-Behandlungseinheit erzeugten Niederfrequenz entspricht, umfasst.

7. Gerät nach Anspruch 1, das weiterhin
einen Hauptnetzschalter zur Wahl der Hautwiderstandsmessung oder der Niederfrequenzstrombehandlung umfasst.

## Revendications

1. Appareil multifonctionnel de manuthérapie Koryo comprenant :
une unité de mémoire pour stocker des informations de points d'acuponcture importants et d'un guide de prescription de manuthérapie ;
une unité d'entrée de touche pour générer des signaux de touche en fonction de la sélection par un utilisateur de la mesure de résistance de la peau, du guide des points d'acuponcture importants et de prescription de la manuthérapie de traitement basse fréquence, et de la thérapie Yeoampa ;
une unité de mesure de résistance de la peau pour délivrer du courant pour mesurer la résistance de la peau, en fonction de la sélection de la mesure de résistance de la peau à partir de l'unité d'entrée de touche, et pour mesurer la résistance de la peau d'un corps sur la base de la valeur d'un résultat ;
une unité de traitement à courant basse fréquence pour fournir une basse fréquence à des points de stimulation d'un corps afin de soulager des douleurs par stimulation de la peau, en fonction de la sélection du traitement basse fréquence à partir de l'unité d'entrée de touche ;
une unité de commande pour extraire des informations correspondantes à partir des informations de points d'acuponcture importants et d'un guide de prescription de la manuthérapie, stockées dans l'unité de mémoire, en fonction du signal de touche généré par l'unité d'entrée de touche, exécuter la thérapie Yeoampa en délivrant un modèle d'acuponcture par manuthérapie pour exécuter la thérapie Yeoampa, et commander des opérations respectivement de l'unité de mesure de résistance de la peau et de l'unité de traitement à courant basse fréquence ; et
une unité d'affichage pour afficher les informations de points d'acuponcture importants et d'un guide de prescription de la manuthérapie délivrées par l'unité de commande, et le modèle d'acuponcture par manuthérapie pour exécuter la thérapie Yeoampa,
appareil dans lequel, pendant l'exécution de la thérapie Yeoampa par l'unité de commande, l'unité de traitement à courant basse fréquence applique un courant basse fréquence à des parties d'un corps qui correspondent au modèle d'acuponcture par manuthérapie pour exécuter la thérapie Yeoampa en fonction des signaux de touche émis par l'unité d'entrée de touche.

2. Appareil selon la revendication 1, dans lequel l'unité de mesure de résistance de la peau comprend :
un compteur de l'unité de mesure pour faire passer le courant dans une partie mesurée, et pour mesurer la résistance de la peau en fonction de la quantité de courant ;
un port de connexion d'un fil d'électrode de mesure des méridiens, qui opère en tant que port pour connecter un fil d'électrode de résistance de la peau ;
un contrôleur du compteur de l'unité de mesure pour commander l'appareil de telle sorte qu'une valeur du compteur devienne une certaine valeur par le contact avec les fils d'électrode de résistance de la peau ; et
une lampe qui s'allume pour indiquer la mesure de la résistance de la peau.

3. Appareil selon la revendication 1, dans lequel l'unité de traitement à courant basse fréquence comprend :
un générateur basse fréquence pour générer une basse fréquence ;
un dispositif de réglage d'intensité de courant pour régler l'intensité du courant de la basse fréquence générée par le générateur basse fréquence ;
un dispositif de réglage de fréquence pour régler la basse fréquence générée par le générateur basse fréquence à une fréquence appropriée ;
une lampe de traitement pour allumer et éteindre la lampe dont la basse fréquence est générée par le générateur basse fréquence ;
une minuterie pour régler un temps de sortie de la basse fréquence générée par le générateur basse fréquence ;
une touche de démarrage pour générer un signal de touche permettant de démarrer le traitement à basse fréquence dans le temps réglé par la minuterie ; et
un port de connexion du fil d'électrode basse fréquence pour connecter un fil d'électrode basse fréquence.

4. Appareil selon la revendication 1, comprenant, en outre :
un contrôleur à distance pour générer des signaux de commande à distance en fonction de la sélection par l'utilisateur du guide des points d'acuponcture importants de la manuthérapie, de la thérapie Yeoampa, de la mesure de résistance de la peau, et du traitement à courant basse fréquence ; et
un récepteur de signaux de commande à distance pour recevoir des signaux de commande à distance du contrôleur à distance.

5. Appareil selon la revendication 1, comprenant, en outre :
une diode électroluminescente pour afficher la résistance de la peau mesurée par l'unité de mesure de résistance de la peau.

6. Appareil selon la revendication 1, comprenant, en outre :
un contrôleur de son pour générer un son aigu à un point de crête lorsque l'unité de mesure de résistance de la peau est en fonctionnement, et pour générer et commander un son correspondant à la basse fréquence générée par l'unité de traitement à courant basse fréquence.

7. Appareil selon la revendication 1, comprenant, en outre :
un commutateur d'alimentation principal pour sélectionner la mesure de résistance de la peau ou le traitement à courant basse fréquence.
